# NOUVEAU FASCICULE DE BREVET EUROPEEN

(11) **EP 0 572 636 B2**
(45) Date de publication et mention de la décision concernant l'opposition: **07.01.2009**
(45) Mention de la délivrance du brevet: 17.04.1996
(21) Numéro de dépôt: 93901792.7
(22) Date de dépôt: 20.11.1992
(51) Int. Cl.: A61Q 5/06, A61K 8/04, A61K 8/34, A61K 8/37, A61K 8/81

(54) **LAQUE AEROSOL CAPILLAIRE**
AEROSOL-HAARSPRAY
AEROSOL HAIR SPRAY

(30) Priorité: 21.11.1991 FR 9114353
(43) Date de publication de la demande: 08.12.1993
(73) Titulaire: L'ORÉAL, 75008 Paris (FR)
(72) Inventeur: STURLA, Jean-Michel, F-92210 Saint-Cloud (FR); BEITONE, Régis, F-75013 Paris (FR)
(74) Mandataire: Le Coupanec, Pascale A.M.P.
(86) Numéro de dépôt international: PCT/FR1992/001081
(87) Numéro de publication internationale: WO 1993/009757

(56) Documents cités:
- EP-A1- 0 418 676
- US-A- 5 053 218
- SEIFEN, OLE, FETTE, WACHSE vol. 117, no. 13, 28 Août 1991, AUGSBURG pages 464 - 467 GUTH. J ET AL. 'Addressing the North American trend toward low VOC hair sprays'
- J. Guth et al.; "Addressing the North American Trend Toward Low VOC Hair Sprays", SÖFW, Vol.117, n°13, pages 462, 464-467.

## Description

La présente invention a pour objet une composition cosmétique aqueuse sous forme d'une laque aérosol pour la fixation des cheveux et plus particulièrement une laque aérosol présentant un pouvoir laquant élevé.

La plupart des laques aérosols jusqu'ici commercialisées sont essentiellement constituées d'un "jus" comprenant une résine filmogène en solution alcoolique contenant éventuellement divers ingrédients cosmétiques tels que des agents plastifiants, des agents adoucissantes, des parfums, des huiles, des lubrifiants, de la lanoline, des silicones, des filtres solaires ainsi que des colorants. Le "jus" est ensuite conditionné dans un récipient aérosol et mis sous pression à l'aide d'un gaz propulseur chlorofluorocarboné tel que les produits vendus sous la dénomination de "Fréons".

Pour des raisons écologiques, la tendance actuelle consiste à remplacer systématiquement dans les récipients aérosols, les gaz propulseurs chlorofluorocarbonés par des propulseurs présentant moins de risque pour la couche d'ozone comme par exemple certains hydrocarbures tels que le propane, le n-butane, l'isobutane ou leurs mélanges en particulier des mélanges de propane-butane ou encore le diméthyléther ou un mélange de ces gaz propulseurs.

Pour d'autres raisons, on a par ailleurs cherché à remplacer totalement ou partiellement le solvant du polymère filmogène par de l'eau. Les résultats n'ont pas été particulièrement convaincants dans la mesure où le pouvoir laquant n'était pas totalement satisfaisant.

Il en est ainsi des compositions aqueuses pour la fixation des cheveux décrites dans US-5.053.218 qui comprennent une résine filmogène naturelle du type shellac, du diméthyléther en tant qu'agent propulseur, de l'eau en tant que solvant et, éventuellement, un agent plastifiant.

Après d'importantes recherches, on visent maintenant de constater qu'il était possible d'obtenir des laques aérosols aqueuses pour cheveux présentant de bonnes propriétés cosmétiques, à savoir notamment un excellent pouvoir laquant.

On entend par "pouvoir laquant" l'aptitude de la laque à maintenir les cheveux dans la forme qu'on leur a conférée. Selon l'invention, ces résultats sont essentiellement obtenus par l'emploi de certains agents plastifiants et de certains polymères filmogènes ainsi que par l'emploi de diméthyléther en tant qu'agent propulseur.

La présente invention a donc pour objet une composition cosmétique aqueuse sous forme d'une laque aérosol telle que définie en revendication 1.

La résine filmogène doit être soluble dans l'eau ainsi que dans les mélanges eau-diméthyléther.

Parmi les résines filmogènes synthétiques utilisables selon l'invention, on peut en particulier citer:
- les terpolymères acétate de vinyle/tert-butylbenzoate de vinyle/acide crotonique décrits dans le brevet français 78.30596 (2.439.798).

Selon une forme de réalisation particulière, les résines filmogènes synthétiques peuvent être utilisées sous forme d'un mélange.

Parmi les résines synthétiques énumérées ci-dessus, celles ayant conduit à des résultats particulièrement intéressants sont celles décrites dans le brevet français 78.30596 (2.439.798).

Les copolymères ci-dessus comportant des motifs d'acide crotonique sont généralement utilisés sous leur forme, partiellement ou totalement, neutralisée à l'aide de soude ou de potasse ou encore à l'aide d'une alcanolamine telle que l'amino-2 méthyl-2 propanol-1 (AMP).

Le copolymère ayant conduit à des laques aérosols particulièrement satisfaisantes est le terpolymère acétate de vinyle/tert-butylbenzoate de vinyle/acide crotonique (65/25/10) dont les fonctions acides sont neutralisées à l'aide d'une alcanolamine et notamment l'AMP.

L'agent plastifiant de la composition aérosol selon l'invention est présent en une proportion comprise entre 20 et 50 % en poids par rapport au poids de la résine.

Tout comme la résine filmogène, l'agent plastifiant doit être soluble dans l'eau et dans les mélanges eau-diméthyléther. Par ailleurs, l'agent plastifiant doit être compatible avec la résine, c'est-à-dire qu'il doit pouvoir s'insérer moléculairement dans le film de résine sans excuder.

Parmi les agents plastifiants particulièrement préférés selon l'invention, on peut notamment citer :
- les éthers de glycol décrits dans l'ouvrage "Industrial Solvents Handbook - Noyes Data Corporation, 2ème Ed., 1977 - pp.346 et suivantes, comprenant les Carbitols vendus par la Société UNION CARBIDE, les Cellosolves vendus par la Société UNION CARBIDE et les Dowanols vendus par la Société DOW CHEMICAL

Parmi les Carbitols, on peut citer: le Carbitol ou diéthylène glycol éthyléther, le méthylCarbitol ou diéthylène glycol méthyléther, le butylCarbitol ou diéthylène glycol butyléther ou encore l'hexylCarbitol ou diéthylèneglycol hexyléther.

Parmi les Cellosolves, on peut citer : le Cellosolve ou éthylène glycol éthyléther, le butylCellosolve ou éthylène glycol butyléther, ou encore l'hexylCellosolve ou éthylèneglycol hexyléther.

Parmi les Dowanols, on peut citer : le Dowanol PM ou propylène glycol méthyléther, le Dowanol DPM ou dipropylène glycol méthyléther, le Dowanol TPM ou tripropylène glycol méthyléther ou encore le Dowanol DM ou diéthylène glycol méthyléther.
- l'huile de ricin oxyéthylénée à 40 moles d'oxyde d'éthylène telle que celle vendue sous la dénomination de MUL-GOFEN EL-719" par la Société RHONE POULENC.
- l'alcool benzylique,
- les citrates d'alkyle et leurs dérivés acétylés vendus sous les dénominations de "CITROFLEX" par la Société MOR-FLEX et en particulier le citrate de triéthyle ("CITROFLEX-2") et l'acétyl citrate de tributyle ("CITROFLEX-A4"),
- le 1,3-butylène glycol,
- le carbonate de propylène,
- le diéthanolamide de l'acide laurique vendu sous la dénomination de "MONAMID 716" par la Société MONA INDUSTRIES, et
leurs mélanges.

Selon un mode de réalisation particulier de l'invention, l'agent plastifiant est de préférence un Dowanol, notamment le Dowanol PM, le Dowanol DPM et plus particulièrement le Dowanol TPM.

Le solvant, à savoir l'eau, est présent dans la composition aérosol en une proportion comprise entre 50 et 70 % en poids par rapport au poids total de la composition.

L'agent propulseur, à savoir le diméthyléther, est présent en une proportion comprise entre 20 et 40 % en poids par rapport au poids total de la composition.

Bien que l'invention ne soit pas limitée à une teneur précise en diméthyléther, on préfère cependant selon l'invention que cette quantité soit telle qu'elle conduise à un mélange liquide homogène.

Selon l'invention, il est souhaitable que dans le récipient aérosol,la pression de vapeur dans la bombe aérosol soit comprise entre environ 2 et 5 bars à 20°C.

Les laques aérosols selon l'invention peuvent bien entendu contenir d'autres ingrédients conventionnels tels que des agents adoucissants, des parfums, des silicones, des filtres solaires, des colorants, des conservateurs, des agents anti-mousse, des vitamines ainsi que des protéines.

Le récipient aérosol dans lequel la composition est conditionnée est de type classique mais peut être éventuellement équipé d'une prise de gaz additionnelle en vue d'obtenir une pulvérisation de qualité plus fine.

On va maintenant donner à titre d'illustration et sans aucun caractère limitatif, plusieurs exemples de laques aérosols selon l'invention.

### EXEMPLE 1 :

On prépare une laque aérosol pour cheveux en conditionnant, dans un récipient aérosol approprié, 6 g du terpolymère acétate de vinyle/tert-butylbenzoate de vinyle/acide crotonique (65/25/10 en poids) selon le brevet français n° 78.30596 (2.439.798), que l'on neutralise à 100 % (d'après l'indice d'acide) par addition de l'amino-2 méthyl-2 propanol-1 (AMP), 3 g de "DOWANOL TPM" et la quantité suffisante d'eau pour compléter à 70 g.

On introduit ensuite selon les techniques conventionnelles, 30 g de diméthyléther et on procède à la fixation de la valve et à la fermeture hermétique du récipient (pression 4,6 bars).

La valve peut être du type avec prise de gaz additionnelle en vue d'obtenir une pulvérisation de qualité plus fine.

Par application de la laque sur des cheveux naturels ou des cheveux sensibilisés, on constate qu'elle a un excellent pouvoir laquant et ne provoque aucun effet de poissage lors de l'application et après.séchage.

### EXEMPLE 2:

Selon le même mode opératoire que celui décrit à l'exemple 1, on a préparé une laque aérosol capillaire ayant la composition suivante :

| | | |
|---|---|---|
| - Terpolymère acétate de vinyle/tert-butylbenzoate de vinyle/acide crotonique (65/25/10) décrit dans le brevet français n°78.30596 (2.439.798) | 6 g | |
| - AMP qs pour neutralisation 100 % | | |
| - "Dowanol TPM" | 1,8 g | |
| - Diméthyléther | 30 g | |
| - Eau | qsp | 100 g |

### EXEMPLE 3:

Selon le même mode opératoire que celui décrit à l'Exemple 1, on a préparé une laque aérosol capillaire ayant la composition suivante :

| | | |
|---|---|---|
| - Terpolymère acétate de vinyle/tert-butylbenzoate de vinyle/acide crotonique (65/25/10) décrit dans le brevet français n° 78.30596 (2.439.798) | 3 g | |
| - AMP qs pour neutralisation 100 % | | |
| - "Dowanol DPM" | 0,9 g | |
| - Diméthyléther | 35 g | |
| - Parfum qs | | |
| - Eau | qsp | 100 g |

## Revendications

1. Composition cosmétique aqueuse sous forme d'une laque aérosol, **caractérisée par le fait qu'**elle contient :
a) entre 3 et 10 % en poids par rapport au poids total de la composition d'au moins une résine filmogène synthétique,
b) entre 20 et 50 % en poids par rapport au poids de la résine d'au moins un agent plastifiant,
c) entre 50 et 70 % en poids d'eau par rapport au fond total de la composition dans laquelle ladite résine et l'agent plastifiant sont à l'état soluble et homogène,
d) entre 20 et 40 % en poids par rapport au poids total de la composition de diméthyléther en tant qu'agent propulseur,
avec ladite résine filmogène, étant choisie parmi :
- les terpolymères acétate de vinyle/tert-butylbenzoate de vinyle/acide crotonique,
- leurs mélanges, et ledit agent plastifiant, étant choisi parmi :
- les éthers de glycol,
- l'huile de ricin oxyéthylénée à 40 moles d'oxyde d'éthylène,
- l'alcool benzylique,
- les citrates d'alkyle et leurs dérivés acétylés,
- la 1,3-butylène glycol,
- la carbonate de propylène,
- la diéthanolamide de l'acide laurique, et
- leurs mélanges.

2. Composition, selon la revendication 1, **caractérisée par le fait que** les polymères comportant des motifs d'acide acrylique ou d'acide crotonique sont neutralisés partiellement ou totalement à l'aide de soude, de potasse ou d'une alcanolamine.

3. Composition selon l'une quelconque des revendications précédentes, **caractérisée par le fait que** la résine synthétique est le terpolymère acétate de vinyle/tert-butylbenzoate de vinyle/acide crotonique (65/25/10) dont les fonctions acides sont neutralisées à l'aide d'une alcanolamine, de préférence l'amino-2 méthyl-2 propanol-1.

4. Composition selon la revendication 1, **caractérisée par le fait que** l'éther de glycol est le tripropylène glycol méthyléther.

5. Composition selon l'une quelconque des revendications précédentes, **caractérisée par le fait qu'**elle contient en outre un agent adoucissant, un parfum, une silicone, un filtre solaire, un colorant, un conservateur, un agent anti-mousse, une vitamine ou une protéine.

## Claims

1. Aqueous cosmetic composition in the form of an aerosol lacquer, **characterized in that** it contains:
a) between 3% and 10% by weight, relative to the total weight of the composition, of at least one synthetic film-forming resin,
b) between 20% and 50% by weight, relative to the weight of the resin, of at least one plasticizer,
c) between 50% and 70% by weight of water, relative to the total weight of the composition, in which the said resin and the plasticizer are in the soluble and homogeneous form,
d) between 20% and 40% by weight, relative to the total weight of the composition, of dimethyl ether as propellant, with
the said film-forming resin being chosen from:
- vinyl acetate/vinyl tert-butylbenzoate/crotonic acid terpolymers, and
- mixtures thereof, and the said plasticizer being chosen from:
- glycol ethers,
- castor oil oxyethylenated with 40 mol of ethylene oxide,
- benzyl alcohol,
- alkyl citrates and acetylated derivatives thereof,
- 1,3-butylene glycol,
- propylene carbonate,
- lauric acid diethanolamide, and
- mixtures thereof.

2. Composition according to Claim 1, **characterized in that** the polymers containing acrylic acid units or crotonic acid units are partially or totally neutralized using sodium hydroxide, potassium hydroxide or an alkanolamine.

3. Composition according to either one of the preceding claims, **characterized in that** the synthetic resin is the vinyl acetate/vinyl tert-butylbenzoate/crotonic acid terpolymer (65/25/10) whose acidic functions are neutralized using an alkanolamine, preferably 2-amino-2-methyl-1-propanol.

4. Composition according to Claim 1, **characterized in that** the glycol ether is tripropylene glycol methyl ether.

5. Composition according to any one of the preceding claims, **characterized in that** it also contains a softener, a fragrance, a silicone, a sunscreen, a dye, a preserving agent, an anti-foaming agent, a vitamin or a protein.

## Patentansprüche

1. Kosmetische wässrige Zubereitung in Form eines Lackaerosols, **dadurch gekennzeichnet, dass** sie
(a) zwischen 3 und 10 Gew.-%, in Bezug auf das Gesamtgewicht der Zubereitung, von mindestens einem synthetischen filmbildenden Harz,
(b) zwischen 20 und 50 Gew.-%, in Bezug auf das Gewicht des Harzes, von mindestens einem Weichmacher,
(c) zwischen 50 und 70 Gew.-%, in Bezug auf das Gesamtgewicht der Zubereitung, an Wasser, worin das Harz und der Weichmacher in gelöstem und homogenem Zustand vorhanden sind,
(d) zwischen 20 und 40 Gew.-%, in Bezug auf das Gesamtgewicht der Zubereitung, an Dimethylether als Treibmittel,
enthält,
wobei das filmbildende Harz ausgewählt ist aus:
- Terpolymeren aus Vinylacetat/Vinyl-tert-Butylbenzoat/Crotonsäure, und
- ihren Gemischen, und
wobei der Weichmacher ausgewählt ist aus:
- Glykolethern
- mittels 40 Mol Ethylenoxid oxyethyliertem Rizinusöl,
- Benzylalkohol,
- Alkylcitraten sowie deren acetylierten Derivaten,
- 1,3-Buylenglykol,
- Propylencarbonat,
- Laurinsäurediethanolamid sowie
- deren Gemischen.

2. Zubereitung nach Anspruch 1, **dadurch gekennzeichnet, dass** die Polymere, die Acrylsäure- oder Crotonsäureeinheiten umfassen, teilweise oder völlig mit Hilfe von Natriumcarbonat, Kaliumcarbonat oder eines Alkanolamins neutralisiert sind.

3. Zubereitung nach einem der vorstehenden Ansprüche, **dadurch gekennzeichnet, dass** das synthetische Harz das Terpolymer aus Vinylacetat/Vinyl-tert-Butylbenzoat/Crotonsäure (65/25/10) ist, dessen saure Funktionen mit Hilfe eines Alkanolamins, vorzugsweise mittels des 2-Amino-2-methyl-1-propanols, neutralisiert sind.

4. Zubereitung nach Anspruch 1, **dadurch gekennzeichnet, dass** der Glykolether Tripropylenglykolmethylether ist.

5. Zubereitung nach einem der vorstehenden Ansprüche, **dadurch gekennzeichnet, dass** sie zusätzlich noch ein Erweichungsmittel, einen Duftstoff, ein Silikon, eine Sonnenfiltersubstanz, einen Farbstoff, ein Konservierungsmittel, ein Antischaummittel, ein Vitamin oder ein Protein enthält.
